(19) **Europäisches Patentamt**

**European Patent Office**

**Office européen des brevets**

(11) **EP 1 283 714 B1**

(12) **FASCICULE DE BREVET EUROPEEN**

(45) Date de publication et mention
de la délivrance du brevet:
**05.07.2006 Bulletin 2006/27**

(51) Int Cl.:
*A61K 35/74* *(2006.01)*     *A23C 9/123* *(2006.01)*
*A61P 31/16* *(2006.01)*

(21) Numéro de dépôt: **01929743.1**

(22) Date de dépôt: **27.04.2001**

(86) Numéro de dépôt international:
**PCT/FR2001/001310**

(87) Numéro de publication internationale:
**WO 2001/089541 (29.11.2001 Gazette 2001/48)**

(54) **UTILISATION DE LACTOBACILLUS CASEI DANS DES COMPOSITIONS IMMUNOSTIMULANTES**

VERWENDUNG VON LACTOBACILLUS CASEI IN IMMUNVERSTÄRKENDEN
ZUSAMMENSETZUNGEN

USE OF LACTOBACILLUS CASEI IN IMMUNOSTIMULATORY COMPOSITIONS

(84) Etats contractants désignés:
**AT BE CH CY DE DK ES FI FR GB GR IE IT LI LU
MC NL PT SE TR**
Etats d'extension désignés:
**RO SI**

(30) Priorité: **25.05.2000 FR 0006679**

(43) Date de publication de la demande:
**19.02.2003 Bulletin 2003/08**

(73) Titulaire: **COMPAGNIE GERVAIS-DANONE
F-92302 Levallois Perret (FR)**

(72) Inventeurs:
• **POSTAIRE, Eric
F-92170 Vanves (FR)**
• **BONAVIDA, Benjamin
Los Angeles, CA 90034 (US)**

(74) Mandataire: **Vialle-Presles, Marie José et al
Cabinet ORES,
36,rue de St Pétersbourg
75008 Paris (FR)**

(56) Documents cités:
**WO-A-96/20607          WO-A-99/10476**

• YASUI H ET AL: "Immunomodulatory function of
lactic acid bacteria." ANTONIE VAN
LEEUWENHOEK, vol. 76, no. 1-4, juillet 1999
(1999-07), pages 383-389, XP000979552
• POUWELS P H ET AL: "The potential of
Lactobacillus as a carrier for oral immunization"
JOURNAL OF BIOTECHNOLOGY, vol. 44, no. 1,
26 janvier 1996 (1996-01-26), pages 183-192,
XP004036864
• UCHIDA S ET AL: "Inhibition of animal virus
plaque formation by culture filtrates of lactic acid
bacteria" KOBE JOURNAL OF MEDICAL
SCIENCES, vol. 24, no. 2, 1978, pages 91-98,
XP000979543
• HORI T ET AL: "Effect of intranasal administration
of Lactobacillus casei Shirota on influenza virus
infection of upper respiratory tract in mice."
CLINICAL AND DIAGNOSTIC LABORATORY
IMMUNOLOGY, vol. 8, no. 3, mai 2001 (2001-05),
pages 593-597, XP001016238

**Description**

L'invention est relative à l'utilisation de bactéries lactiques pour potentialiser la réponse immune spécifique vis-à-vis d'un agent infectieux des voies respiratoires.

Les bactéries lactiques (LAB) sont traditionnellement utilisées pour fabriquer des produits alimentaires fermentés, en particulier des produits laitiers.

Les effets des bactéries lactiques sur la santé ont été suggérés initialement par les travaux de Metchnikoff (The prolongation of life. 1st ed. New York: GP Putman's Sons, 1908), et ont depuis fait l'objet de nombreuses recherches.

Il est maintenant généralement reconnu que diverses bactéries lactiques peuvent exercer un rôle bénéfique pour la santé. Ces bactéries sont également dénommées « probiotiques », appellation qui désigne des microorganismes vivants qui, lorsqu'il sont ingérés en quantité suffisante, exercent un effet positif sur la santé, au-delà des effets nutritionnels traditionnels. Des bactéries probiotiques ont notamment été décrites parmi des espèces appartenant aux genres *Lactobacillus, Bifidobacterium, Streptococcus* et *Lactococcus,* couramment utilisés dans l'industrie laitière.

Les probiotiques interviendraient notamment au niveau de la flore intestinale en freinant le développement des microorganismes pathogènes, et/ou en agissant plus directement sur le système immunitaire. Il a par exemple été observé que l'ingestion de bactéries probiotiques ou d'aliments fermentés, tels que le yoghourt, comprenant ces bactéries, entraînait une diminution des bactéries pathogènes ; au niveau du système immunitaire, différents effets ont été rapportés : une activation des cellules intervenant dans la réponse immune spécifique ou non-spécifique, telles que les lymphocytes et les macrophages, une augmentation du taux d'immunoglobulines et notamment des IgA ; une augmentation du taux de cytokines activatrices du système immunitaire, etc. (pour revue, cf. par exemple MEYDANI et HA (Am J Clin Nutr, 71, 861-7217, 2000).

Dans leur ensemble, les études effectuées sur différentes bactéries lactiques probiotiques tendent à conclure à l'existence des propriétés immunostimulantes de certaines espèces, ou du moins de certaines souches de ces espèces. En revanche, le ou les mécanisme(s) sous-tendant ces propriétés, et les composantes du système immunitaire potentiellement impliquées, demeurent incertains. Il apparaît donc nécessaire d'élucider ces aspects, notamment afin de proposer des applications mieux ciblées des différentes espèces ou souches de bactéries probiotiques.

Plusieurs études effectuées chez l'Homme et l'animal suggèrent un effet salutaire sur la santé de bactéries de l'espèce *L. casei,* et en particulier un effet positif sur le système immunitaire.

Ainsi, il a été montré chez la souris que l'ingestion de lait fermenté contenant la souche DN-114 001 de *L. casei* accroissait la résistance à une infection par *Salmonella typhimurium* [PAUBERT-BRAQUET et al. Int J Immunother, 4:153, (1995) ; une activation des macrophages et une augmentation des IgA circulantes ont été observées simultanément.

La souche DN-114 001 a été déposée le 30 décembre 1994, auprès de la CNCM (Collection Nationale de Cultures de Microorganismes) tenue par l'Institut Pasteur, 25 rue du Docteur Roux, à Paris, sous le numéro I-1518 ; cette souche, et son association avec des ferments du yogourt pour la préparation de produits laitiers fermentés sont décrits dans la Demande PCT WO 96/20607 au nom de COMPAGNIE GERVAIS DANONE.

Une étude récente effectuée chez l'Homme rapporte également que la consommation de lait fermenté contenant la souche DN-114 001 de *L. casei* induit un renforcement de la résistance à *Salmonella typhimurium.* Cet effet est attribué à une action au niveau de l'immunité innée, non-spécifique [YOON et al., Int J Immunother; 15, 79-89 (1999)].

Les Inventeurs ont recherché si *L. casei* possédait également une action au niveau de l'immunité adaptative, qui contrairement à l'immunité innée, se traduit par une réponse immune spécifique vis-à-vis d'un agent pathogène donné.

Dans ce but, les Inventeurs ont étudié l'effet de *L. casei* administré oralement in vivo, sur la stimulation ex vivo de la prolifération des cellules T en réponse à des antigènes représentatifs de différents types d'agents pathogènes communs : un antigène bactérien, (tétanos) ; un antigène fongique (candida) et un antigène viral (grippe).

Ils ont observé, pour chacun des antigènes testés, que l'ingestion de *L. casei* entraînait une augmentation de la capacité proliférative des cellules T, et notamment des sous-populations CD3$^+$, en réponse à une activation par ledit antigène. Cet effet se manifeste plus particulièrement dans le cas de l'antigène de la grippe.

La présente invention a pour objet l'utilisation d'une souche bactérienne de l'espèce *L. casei* pour la préparation d'une composition administrable par voie orale pour renforcer une réponse immunitaire systémique spécifique vis-à-vis d'un micro-organisme pathogène des voies respiratoires.

Ce renforcement de la réponse immunitaire découle d'une augmentation de la capacité proliférative des cellules T spécifiques des antigènes dudit micro-organisme pathogène.

Les pathogènes concernés sont surtout des bactéries ou des virus ; parmi ces derniers on citera par exemple les rhinovirus, le virus respiratoire syncitial (V.R.S.), et les myxovirus (orthomyxovirus tels que les virus de la grippe (influenza type A, B, ou C), ou paramyxovirus, et notamment para-influenzae).

Dans le cadre de la mise en oeuvre de la présente invention, ladite souche de *L. casei* peut être utilisée seule ou en association avec d'autres bactéries lactiques de l'espèce *L. casei* ou d'autres espèces. Avantageusement, elle peut être utilisée en association avec des ferments du yogourt, à savoir *Lactobacillus bulgaricus* et *Streptococcus*

*thermophilus.*

Elle peut être utilisée sous forme de bactéries entières, vivantes ou non, et aussi sous forme de lysat bactérien, ou sous forme de fractions bactériennes.

De préférence, une composition préparée dans le cadre d'une utilisation conforme à l'Invention contient au moins $10^5$, de préférence au moins $10^6$, généralement entre $1 \times 10^8$ et $1,5 \times 10^9$ cellules de *L. casei* par ml.

Lorsque *L. casei* est utilisé en association avec des ferments du yogourt, ladite composition comprend en outre avantageusement au moins $10^7$, de préférence entre $2 \times 10^8$ et $1 \times 10^9$ cellules de *S. thermophilus* par ml, et au moins $5 \times 10^5$ et de préférence entre $4 \times 10^6$ et $2 \times 10^7$ cellules de *L. bulgaricus* par ml.

Une souche de *L. casei* convenant tout particulièrement à une utilisation dans la présente invention est la souche CNCM I-1518.

Des compositions préparées conformément à l'invention peuvent être administrées sous forme d'aliments ou de compléments alimentaires. Il peut s'agir par exemple de produits laitiers, et notamment de produits laitiers fermentés comprenant au moins ladite souche de *L. casei,* éventuellement associée à d'autres bactéries lactiques, par exemple à des ferments du yogourt.

Des compositions préparées conformément à la présente invention sont utilisables dans le cadre de la prévention et du traitement de pathologies d'origine infectieuse, et notamment d'origine virale, et en particulier de la grippe. De préférence, pour obtenir un effet optimal elles seront administrées pendant au moins une semaine, et avantageusement pendant au moins 10 jours, en quantité correspondant à l'absorption d'au moins $10^7$, de préférence au moins $10^8$, généralement entre $10^9$ et $10^{12}$ cellules de *L. casei.*

La présente invention sera mieux comprise à l'aide du complément de description qui va suivre, qui se réfère à des exemples illustrant les propriétés d'une souche de *Lactobacillus casei* pour renforcer la réponse spécifique à des antigènes microbiens.

**EXEMPLE 1 : ACTION DE *LACTOBACILLUS CASEI* SUR LA PROLIFÉRATION DES CELLULES T EN REPONSE A UNE STIMULATION ANTIGENIQUE**

Une étude en double aveugle contre placebo a été effectuée pour tester l'effet de l'ingestion d'un produit laitier fermenté comprenant la souche DN-114 001 (CNCM I-1518) de *Lactobacillus casei* sur la prolifération des cellules T en réponse à une stimulation antigénique.

Les conditions de cette étude sont les suivantes :

Sujets

88 sujets sains âgés de 18 à 50 ans ont été recrutés dans le Département des Maladies Infectieuses, Washington Hospital Center, (Washington DC). Les sujets présentant des antécédents d'hépatite ou de troubles rénaux, de dysfonctionnements cardio-vasculaires, de maladies immunitaires ou gastro-intestinales, d'asthme sévère, de diabète sucré, les sujets ayant suivi un traitement antibiotique ou immunosuppresseur moins de 3 mois avant le commencement de l'étude, les sujets présentant des antécédents d'abus ou de dépendance à l'alcool, les sujets présentant une intolérance ou une hypersensibilité connue aux produits laitiers, les sujets soumis à un régime hypocalorique, les sujets ayant été vaccinés contre la grippe lors de la saison précédente, ainsi que les femmes enceintes ou allaitantes ont été exclus de l'étude.

Le protocole et les conditions de l'étude ont été approuvées par le Comité de Recherche du WHC, ainsi que par le Comité de Révision Institutionnel qui contrôle les études effectuées chez l'Homme.

Les sujets retenus ont été répartis aléatoirement en 2 groupes :

- un groupe de 47 sujets (26 femmes et 21 hommes), a reçu 100 ml par jour d'un produit laitier fermenté à 2% de matière grasse, comprenant des ferments du yogourt (L. *bulgaricus* and S. *thermophilus)* et la souche DN-114 001 de *Lactobacillus casei ,* et commercialisé par DANONE sous la marque ACTIMEL.
- un groupe de 41 sujets (22 femmes et 19 hommes) a reçu pendant 28 jours un placebo : 100 ml par jour de lait dilué avec de l'eau (1/5, v/v) additionné de sucre pour lui conférer une valeur calorique équivalente à celle de l'ACTIMEL.

Il a été demandé aux sujets des 2 groupes de s'abstenir de consommer du yogourt ou d'autres produits laitiers fermentés pendant la durée de l'étude.

La moyenne (écart-type indiqué entre parenthèses) d'âge des sujets était respectivement de 36 ans (7,3) et de 32,7 ans (7,4) pour le groupe ACTIMEL et le groupe placebo.

Un échantillon de sang (55 ml) a été prélevé chez chaque sujet des 2 groupes avant le début de la consommation d'ACTIMEL ou de placebo, afin de déterminer les valeurs de base. Les sujets ont subi des visites de contrôle aux jours 9, 18, et 28, et ont complété un questionnaire court concernant leur santé, les effets secondaires éventuels, ainsi que le développement de toute maladie pouvant interférer avec l'interprétation des résultats. Un échantillon de sang a été prélevé lors de chaque visite en vue des expérimentations immunologiques.

Pour chaque échantillon de sang individuel, une numération globulaire complète et une analyse de la chimie du sang ont été effectuées. En outre, une analyse phénotypique des leucocytes et de leurs sous-ensembles (cellules T et sous-ensembles, cellules B, monocytes et cellules NK) a été effectuée par cytométrie de flux.

Mesure de la prolifération cellulaire

La réponse proliférative des cellules T des sujets du groupe ACTIMEL et des sujets du groupe placebo vis-à-vis des trois antigènes microbiens : candida, tétanos, et grippe a été déterminée *ex vivo* par mesure de l'incorporation de $^3$HTdR comme décrit ci-après.

*Préparation des cellules :*

30 ml de sang hépariné de chacun des prélèvements effectués ont été traités comme suit : Le tube a été centrifugé pendant 10 min à 1500 tr/min. Le plasma a été transféré avec soin dans des cryotubes étiquetés et entreposé à 70°C. Le sang a été transféré dans un tube de centrifugeuse de 15 ml et dilué au 1:3 avec du PBS 10 ml de Ficoll-hypaque ont été ajoutés dans un tube de centrifugeuse de 50 ml et recouvert de 30 ml du sang dilué. Le tube a été centrifugé pendant 2 min à 2000 tr/min à température ambiante. La couche supérieure de PBS a été aspirée et les cellules mononucléaires ont été rassemblées et transférées dans un tube de centrifugeuse de 15 ml. 10 ml de HBSS ont été ajoutés, vortexés et centrifugés à 1500 tr/min pendant 10 mn ; le surnageant a été aspiré et le culot resuspendu dans 2-3 ml HBSS. 8 ml de HBSS ont été ajoutés et le tube a été centrifugé à 1500 tr/min pendant 6 mn. Ce protocole a été répété 2 fois et les cellules récupérées ont été resuspendues dans 1-2 ml de PBS puis vortexées. Les cellules ont été comptées en examinant leur viabilité par exclusion du Bleu Trypan, et leur concentration a été ajustée à $2 \times 10^8$/ml.

*Préparation des antigènes :*

Antigène du tétanos : la préparation a été faite le jour de l'essai. Une solution mère a été préparée par dilution au 1:1000 de toxoïde tétanique (CONNAUGHT LABORATORY, Willowdale, Canada) dans du PBS.

Antigènes de *Candida albicans :* une solution mère a été préparée par dilution au 1:1000 d'antigènes de *Candida albicans* (BAYER CO, Elkert, IN) dans du PBS.

Antigènes de la grippe : une solution mère a été préparée par dilution au 1:125 d'une préparation d'antigènes du virus de la grippe (NIBSC, Hetz, Angleterre) dans du PBS.

Des plaques de microtitration (96 puits) ont été utilisées. Chaque puits a reçu 100 $\mu$l de solution mère d'antigène et 100 $\mu$l de cellules à une concentration finale de $2 \times 10^5$ cellules par puits. Des puits témoin ont reçu 100 $\mu$l de PBS à la place de la solution mère d'antigène. Les plaques ont été incubées à 37°C, 5% $CO_2$ pendant 5 jours ; 18 heures avant la fin de l'incubation, 20 $\mu$l de 50 $\mu$Ci/ml $^3$HTdR (NEW ENGLAND NUCLEAR, Boston, MA) ont été ajoutés. Les cellules des puits de chaque plaque ont été récoltées sur des filtres et ceux-ci ont été comptés directement dans un compteur bêta MATRIX 9600 (PACKARD INSTRUMENTS) pour mesurer l'incorporation de $^3$HTdR, qui est exprimée en CPM (coups par minute).

La prolifération est évaluée, pour chaque sujet du groupe ayant reçu ACTIMEL et pour chaque sujet du groupe ayant reçu le placebo, en déterminant l'indice de stimulation (IS) selon la formule suivante :

$$IS: = \frac{\text{moyenne CPM cellules stimulées}}{\text{moyenne CPM cellules non-stimulées (puits témoin)}}$$

Analyse statistique

Des statistiques descriptives (moyenne, écart-type, médiane) ont été utilisées pour résumer la réponse proliférative des cellules T à l'exposition à chaque antigène, pour chacun des groupes de l'étude à chaque temps de mesure. Du fait du biais de distribution de la réponse proliférative, la transformation en logarithme naturel a été employée pour les données de prolifération.

Les variations de la réponse proliférative par rapport à la valeur de base ont été utilisées pour la modélisation statistique [LITTELL et al. SAS System for Mixed Model. North Carolina: SAS Institute Inc, (1996)]. Les modifications de la réponse proliférative par rapport à la valeur de base en fonction du temps ont été représentées par technique de lissage de courbes comme décrit par DIGGLE et al. [Analysis of Longitudinal Data. New York: University Press Inc, 1994].

Un modèle linéaire mixte a été développé pour étudier la trajectoire de la réponse proliférative. Ce modèle permet l'ajustement des données de prolifération à une courbe quadratique pour chaque groupe de l'étude.

Ce modèle est défini par l'équation suivante:

$$Y_{ijt} = \alpha_{ij} + \beta 1_j \, T + \beta 2_j \, T^2 + \varepsilon_{ijt}, \quad i=1,\ldots n, \quad j=1, 2, \quad t=1, 2, 3, 4$$

où :

Yijt désigne la variation de la réponse proliférative par rapport à la valeur de base pour le sujet i dans le groupe de produit j au temps t; la transformation en logarithme naturel ayant été appliquée à chaque variable, Yijt = Log (prolifération au temps t) - Log (valeur de base).

$\alpha_{ij}$ désigne l'interception pour le sujet i dans le groupe de produit j. Il reflète un effet aléatoire dans le modèle. $\alpha_{ij} \sim$ MVN (0, G), G contient les composants de la variance en structure diagonale (MVN : distribution variable multinormale).

$\beta 1_j$ and $\beta 2_j$ sont les coefficients de régression de T et $T^2$ pour le groupe de produit j;

T est le moment de la mesure (jour 0, 9, 18, ou 28) et $T^2$ est le terme quadratique de T.

$\varepsilon_{ijt}$ est le coefficient d'erreur :

$\varepsilon_{ijt} \sim$ MVN (0, R) et R=$\sigma^2 I_n$, où $I_n$ désigne la matrice d'identité n x n.

Les tests statistiques pour les hypothèses suivantes ont été effectués:

a) Tester si le coefficient de régression estimé est 0.

$H_0$: $\beta 1_j = 0$ vs. $H_1$: $\beta 1_j \neq 0$ (pour un terme linéaire)

et $H_0$: $\beta 2_j = 0$ vs. $H_1$: $\beta 2_j \neq 0$ (pour un terme quadratique)

Si $\beta 1_j$ et $\beta 2_j$ ne sont pas différents de 0 il n'y a aucune trajectoire.

Si $\beta 2_j$ est significativement différent de 0, il y a une trajectoire quadratique

Si seul $\beta 1_j$ est significativement différent de 0, il y a une trajectoire linéaire.

b) Comparer si les trajectoires des deux groupes sont identiques.

$H_0$: $\beta 1_1 = \beta 1_2$ vs. $H_1$: $\beta 1_1 \neq \beta 1_2$ (pour un terme linéaire)

et $H_0$: $\beta 2_1 = \beta 2_2$ vs. $H_1$: $\beta 2_1 \neq \beta 2_2$ (pour un terme quadratique).

Le progiciel statistique SAS a été utilisé pour effectuer les analyses.

## Résultats

Le tableau I ci-après récapitule les données démographiques concernant les sujets et l'évaluation de la valeur de base de prolifération.

Tableau I

| Données démographiques et valeurs de base | | Placebo | ACTIMEL | comparaison Placebo vs. ACTIMEL |
|---|---|---|---|---|
| Sexe | Femme | 22 | 26 | Test Chi-deux |
| | Homme | 19 | 21 | P=0,826 |
| Age | Moyenne (SD) | 32,7 (7,4) | 36,0 ± 7,3 | T-test |
| | Médiane | 31 | 37 | P=0,038 |
| Ethnie | White | 26 | 25 | Test Chi-deux |
| | Black | 12 | 17 | Exact p-value=0,652 |
| | Other | 3 | 5 | |
| Leucocytes | Moyenne (SD) | 5997 (1451) | 5810 (1399) | T-test |
| | Médiane | 5992 | 5990 | p=0,540 |

Suite de tableau

| Données démographiques et valeurs de base | | Placebo | ACTIMEL | comparaison Placebo vs. ACTIMEL |
|---|---|---|---|---|
| Lymphocytes | Moyenne (SD) Médiane | 1932 (567) 1812 | 2009 (554) 1993 | T-test P=0,519 |
| CD3$^+$CD4$^+$ | Moyenne (SD) Médiane | 865 (327) 740 | 907 (325) 818 | T-test P=0,546 |
| CD3$^+$CD8$^+$ | Moyenne (SD) Médiane | 469 (205) 417 | 431 (181) 403 | T-test P=0,360 |
| CD3$^+$CD25$^+$ | Moyenne (SD) Médiane | 380 (200) 349 | 408 (182) 392 | T-test P=0,489 |
| CD3$^+$CD45$^+$ | Moyenne (SD) Médiane | 1384 (488) 1354 | 1411 (434) 1382 | T-test P=0,783 |
| SD= écart-type | | | | |

Prolifération cellulaire T en réponse à des antigènes microbiens spécifiques

Les moyennes, écarts-types et médianes de la prolifération ex vivo des cellules mononucléées du sang périphérique (PBMC) pour chaque groupe (ACTIMEL et placebo), aux différents temps de prélèvement sont récapitulées dans le Tableau 2 ci-après.

Tableau 2

| Antigène | Placebo | | | | ACTIMEL | | | |
|---|---|---|---|---|---|---|---|---|
| | Jour 0 | Jour 9 | Jour 18 | Jour 28 | Jour 0 | Jour 9 | Jour 18 | Jour 28 |
| Candida Moyenne | 2,3 | 2,7 | 2,9 | 1,9 | 3,0 | 3,2 | 4,3 | 2,3 |
| SD | 2,0 | 3,1 | 4,1 | 1,2 | 3,4 | 3,5 | 5,6 | 2,6 |
| Médiane | 1,5 | 1,7 | 1,6 | 1,6 | 1,7 | 1,7 | 2,0 | 1,4 |
| Tetanos Moyenne | 7,3 | 11,8 | 7,9 | 8,7 | 8,4 | 8,7 | 10,3 | 7,8 |
| SD | 7,8 | 12,3 | 8,0 | 8,1 | 15,8 | 10,4 | 11,8 | 8,2 |
| Médiane | 5,2 | 6,3 | 5,1 | 6,7 | 3,5 | 5,2 | 5,0 | 5,1 |
| Grippe Moyenne | 14,2 | 21,7 | 17,0 | 17,3 | 13,0 | 19,7 | 20,3 | 13,9 |
| SD | 11,4 | 19,1 | 19,6 | 15,7 | 12,6 | 17,1 | 16,6 | 12,7 |
| Médiane | 11,1 | 16,5 | 9,8 | 12,0 | 9,6 | 16,9 | 17,0 | 11,0 |
| SD= ecart-type | | | | | | | | |

Il n'y a pas de différences significatives entre les deux groupes de l'étude pour les valeurs de base (jour 0) de la réponse proliférative vis-à-vis des trois antigènes microbiens.

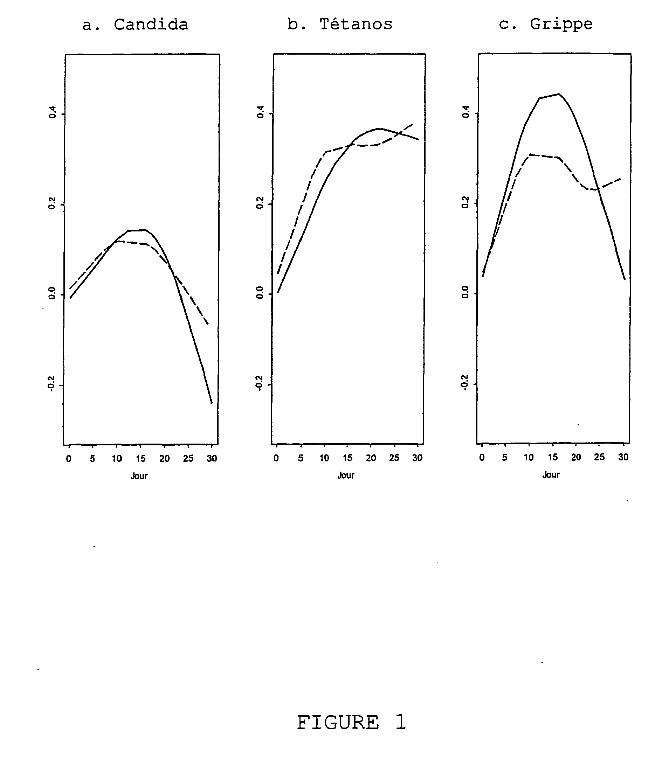

La figure 1 illustre les variations de la réponse proliférative au cours du temps pour chaque antigène pour les deux groupes de l'étude : groupe ACTIMEL : ligne continue ; groupe placebo : ligne pointillée.

Les résultats de la modélisation statistique sont fournis dans le Tableau 3 ci-dessous.

Tableau 3

| Antigène | Variable | | Estimation du paramètre | Erreur Standard | Test $H_0$: paramètre=0 | Comparaison des 2 courbes |
|---|---|---|---|---|---|---|
| Candida | Placebo | T | 0,017 | 0,014 | P=0,232 | $H_0$: $\beta1_1 = \beta1_2$ |
| | | $T^2$ | -0,0007 | 0,0005 | P=0,208 | P=0,540 |
| | ACTIMEL | T | 0,028 | 0,013 | P=0,030 | $H_0$: $\beta2_1 = \beta2_2$ |
| | | $T^2$ | -0,0012 | 0,0005 | P=0,013 | P=0,458 |
| Tetanos | Placebo | T | 0,034 | 0,014 | P=0,022 | $H_0$: $\beta1_1 = \beta1_2$ |
| | | $T^2$ | -0,0008 | 0,0005 | P=0,141 | P=0,630 |
| | ACTIMEL | T | 0,043 | 0,013 | P=0,001 | $H_0$: $\beta2_1 = \beta2_2$ |
| | | $T^2$ | -0,0011 | 0,0005 | P=0,020 | P=0,626 |
| Grippe | Placebo | T | 0,022 | 0,017 | P=0,181 | $H_0$: $\beta1_1 = \beta1_2$ |
| | | $T^2$ | -0,0004 | 0,0006 | P=0,475 | P=0,045 |
| | ACTIMEL | T | 0,068 | 0,015 | P=0,0001 | $H_0$: $\beta2_1 = \beta2_2$ |
| | | $T^2$ | -0,0023 | 0,0006 | P=0,0001 | P=0,027 |

Candida:

Dans le groupe ACTIMEL, la réponse de base $3,0\pm3,4$ au jour 0 augmente à $3,2\pm3,5$ au jour 9, et $4,3\pm5,6$ au jour 18, puis chute à $2,3\pm2,6$ au jour 28 (Table 2). Les coefficients estimés pour le terme linéaire T et le terme quadratique $T^2$ sont 0,028 et 0,0012, respectivement (Table 3). Les deux valeurs sont statistiquement significativement différentes de 0 (p=0,030 et 0,013, respectivement). Ces observations impliquent qu'il y a une tendance positive significative pour la variation de la réponse proliférative à l'antigène de *Candida*. La réponse proliférative augmente d'abord, puis décroît, comme le montre la Figure 1a.

Le groupe du placebo montre un faible changement sur la période de l'étude. Les coefficients de régression estimés pour T et $T^2$ sont de 0,017 et 0,0007. Ces valeurs ne sont pas significativement différentes de 0 (p=0,232 et 0,208, respectivement). Ces observations indiquent qu'il n'y a aucun changement significatif dans la réponse proliférative pour la période de l'étude dans le groupe témoin. Comme le montre la Figure 1a, la courbe pour le groupe placebo est plus plate que la courbe pour le groupe ACTIMEL. Bien que le groupe ACTIMEL montre une trajectoire significative, contrairement au groupe placebo, la différence entre les deux courbes n'atteint pas le niveau de signification statistique (p=0,540 pour T et p=0,458 pour $T^2$, Tableau 3).

Tétanos:

Pour le groupe ACTIMEL, la réponse a augmenté progressivement du niveau de base jusqu'au jour 18, puis a chuté. Les coefficients de régression estimés pour T et $T^2$ sont 0,043 et .0,0011, respectivement (Table 3). Ces deux valeurs sont significativement différentes de 0 (p=0,001 et 0,020, respectivement). Comme dans le cas de *Candida,* la variation positive de la réponse proliférative sur la période de l'étude est significative pour le groupe ACTIMEL. Dans le groupe placebo, la valeur moyenne a augmenté de 7,3 au jour 0 à 11,8 au jour 9, puis est retombée à 7,9 au jour 18, et a augmenté à nouveau à 8,7 au jour 28. (Figure 1b). Même avec un coefficient non-significatif pour le terme du second degré, $T^2$ (-0,0008, p=0,141, Tableau 3) le coefficient estimé significatif de T (0,034, p=0,022, Table 3) montre un effet de trajectoire. Cependant, si l'on compare le groupe ACTIMEL avec le groupe placebo, les tests statistiques ne montrent pas de différences significatives entre les deux courbes (p=0,630 et 0,626 pour T et T2, respectivement).

Grippe:

Le groupe ACTIMEL a montré un changement plus net que le groupe placebo. La valeur moyenne a augmenté de $13\pm12,6$ au jour 0 à $19,7\pm17,1$ au jour 9, est restée à ce niveau au jour 18, et a chuté à $13,9\pm12,7$ au jour 28 (Figure 1c). Les deux coefficients estimés pour T et $T^2$ sont significativement différents de zéro (p=0,001). Ces statistiques indiquent que le changement positif de la réponse proliférative pendant la période de l'étude est significatif (Tableau 2).

Le groupe du placebo a montré des variations semblables à celles observées pour le tétanos. Les valeurs moyennes ont augmenté au jour 9, ont chuté au jour 18, ont encore augmenté au jour 28 comme illustré dans la Figure 1c. Les deux coefficients de régression estimés ne sont pas significativement différents de 0 (les valeurs p sont 0,181 et 0,475 pour T et T2, respectivement), indiquant qu'il n'y a pas de trajectoire significative des variations de la réponse proliférative à l'antigène de la grippe.

Quand on compare les paramètres estimés pour le groupe ACTIMEL et le groupe placebo, des différences significatives sont observées, à la fois pour les paramètres linéaires et les paramètres quadratiques (p=0,045 pour T et p=0,027 pour T2) (Tableau 3). Ces conclusions indiquent que les variations de la réponse proliférative sont significativement différentes entre les deux groupes de l'étude.

**EXEMPLE 2 : ACTION DE *LACTOBACILLUS CASEI* SUR LA PROLIFÉRATION DE SOUS-ENSEMBLES DE LYMPHOCYTES T EN REPONSE A UNE STIMULATION PAR L'ANTIGENE DE LA GRIPPE**

La nature exacte du (des) sous-ensembles T répondant *in vitro* aux antigènes microbiens n'est pas connue. Ou bien chacun des sous-ensembles particuliers de lymphocytes T, tel que ceux représentés par les phénotypes CD3+CD4+, CD3+CD8+, CD3+CD25+, et CD3+CD45+, peut être considéré comme un répondeur primaire, ou bien ces sous-ensembles combinés peuvent participer à la réponse proliférative totale aux antigènes de rappel.

En présupposant que des sous-ensembles particuliers de lymphocytes sont les répondeurs principaux aux antigènes de rappel, des analyses utilisant le modèle statistique décrit à l'exemple 1 ci-dessus ont été effectuées pour les sous-ensembles de cellules T CD3$^+$CD4$^+$, CD3$^+$CD8$^+$, CD3$^+$CD25$^+$, et CD3$^+$CD45$^+$, afin de déterminer si la réponse proliférative obtenue pour chacun des 4 sous-ensembles différait significativement entre le groupe ACTIMEL et le groupe placebo.

Dans ces analyses, les valeurs de la réponse proliférative initialement basées sur les PBMC totales ont été converties pour chaque sous-ensemble en nouvelles valeurs calculées d'après la fréquence totale des cellules T CD3+CD4+, CD3+CD8+, CD3+CD25+, et CD3+CD45+ dans chaque échantillon de sang. Ensuite, des analyses statistiques ont été effectuées pour chaque sous-ensemble, comme décrit à l'exemple 1 ci-dessus.

Les moyennes, écarts types et médianes de chacun des groupes placebo et ACTIMEL, pour les quatre sous-ensembles de lymphocytes sont récapitulés dans le Tableau 4 ci-dessous.

Tableau 4

| Sous-ensemble de Lymphocytes | Placebo | | | | ACTIMEL | | | |
|---|---|---|---|---|---|---|---|---|
| | Jour 0 | Jour 9 | Jour 18 | Jour 28 | Jour 0 | Jour 9 | Jour 18 | Jour 28 |
| CD3+ CD4+ | | | | | | | | |
| Moyenne | 101 | 149 | 113 | 125 | 87,4 | 131 | 133 | 107 |
| SD | 84,0 | 130 | 117 | 127 | 104 | 90,2 | 112 | 127 |
| Médiane | 72,8 | 111 | 74,8 | 91,0 | 63,0 | 103 | 92,0 | 64,5 |
| CD3+ CD8+ | | | | | | | | |
| Moyenne | 192 | 268 | 251 | 251 | 230 | 301 | 365 | 244 |
| SD | 155 | 250 | 289 | 336 | 375 | 286 | 554 | 302 |
| Médiane | 167 | 218 | 140,5 | 168 | 105 | 180 | 214 | 126 |
| CD3+ CD25+ | | | | | | | | |
| Moyenne | 250 | 377 | 260 | 354 | 177 | 267 | 310 | 270 |
| SD | 219 | 374 | 208 | 462 | 139 | 163 | 210 | 305 |
| Médiane | 185 | 263 | 228 | 247 | 159 | 262 | 251 | 177 |
| CD3+ CD45+ | | | | | | | | |
| Moyenne | 63,5 | 87,6 | 73,0 | 76,2 | 52,1 | 78,7 | 87,1 | 69,1 |
| SD | 54,1 | 70,9 | 81,3 | 79,7 | 72,2 | 56,3 | 79,2 | 95,2 |
| Médiane | 47,5 | 66,6 | 43,1 | 51,4 | 34,0 | 64,0 | 67,8 | 41,0 |
| SD= écart type | | | | | | | | |

Les différences des valeurs de base entre les deux groupes de l'étude ne sont pas statistiquement significatives.

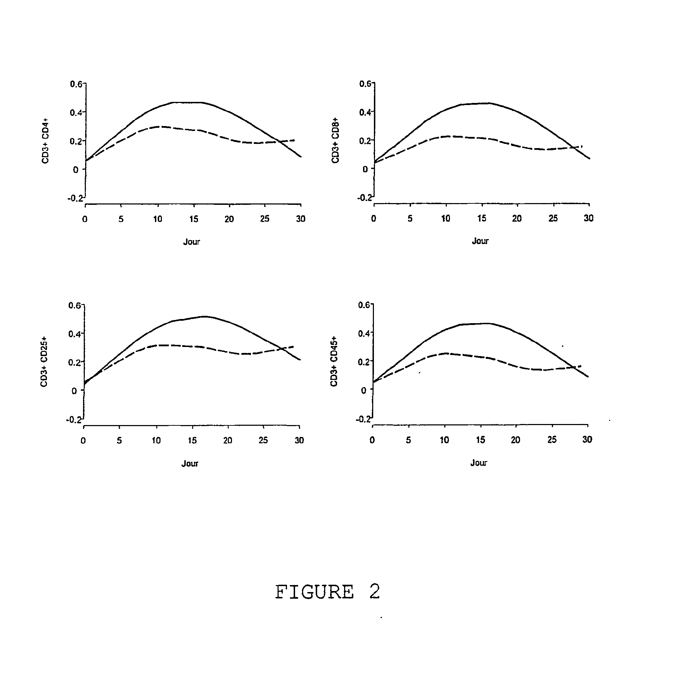

En utilisant le modèle statistique décrit à l'exemple 1 ci-dessus, les variations de la prolifération par rapport à ces valeurs de base en réponse à l'antigène de la grippe ont été analysées pour chacun des quatre sous-) ensembles de lymphocytes. La Figure 2 illustre les variations de la prolifération, en échelle logarithmique, pour chacun de ces sous-ensembles et pour chacun des deux groupes de l'étude.

Les résultats de la modélisation statistique sont récapitulés dans le Tableau 5 ci-dessous.

Tableau 5

| Sous-ensemble de Lymphocytes | Variable | | Estimation du paramètre (SE) | Test $H_o$: paramètre=0 | Comparaison des 2 courbes |
|---|---|---|---|---|---|
| CD3+CD45+ | Placebo | T<br>$T^2$ | 0,015 (0,016)<br>-0,0003 (0,0006) | P=0,352<br>P=0,597 | $H_0$: $\beta1_1 = \beta1_2$<br>P=0,015 |
| | ACTIMEL | T<br>$T^2$ | 0,071 (0,015)<br>-0,0024 (0,0006) | P<0,001<br>P<0,001 | $H_0$: $\beta2_1 = \beta2_2$<br>P=0,016 |
| CD3+CD25+ | Placebo | T<br>$T^2$ | 0,024 (0,016)<br>-0,0004 (0,0006) | P=0,144<br>P=0,463 | $H_0$: $\beta1_1 = \beta1_2$<br>P=0,031 |
| | ACTIMEL | T<br>$T^2$ | 0,071 (0,015)<br>-0,0022 (0,0006) | P<0,001<br>P<0,001 | $H_0$: $\beta2_1 = \beta2_2$<br>P=0,030 |
| CD3+ CD8+ | Placebo | T<br>$T^2$ | 0,014 (0,017)<br>-0,0003 (0,0006) | P=0,410<br>P=0,671 | $H_0$: $\beta1_1 = \beta1_2$<br>P=0,013 |
| | ACTIMEL | T<br>$T^2$ | 0,070 (0,015)<br>-0,0024 (0,0006) | P<0,001<br>P<0,001 | $H_0$: $\beta2_1 = \beta2_2$<br>P=0,012 |
| CD3+ CD4+ | Placebo | T<br>$T^2$ | 0,022 (0,017)<br>-0,0005 (0,0006) | P=0,203<br>P=0,436 | $H_0$: $\beta1_1 = \beta1_2$<br>P=0,028 |
| | ACTIMEL | T<br>$T^2$ | 0,072 (0,015)<br>-0,0025 (0,0006) | P<0,001<br>P<0,001 | $H_0$: $\beta2_1 = \beta2_2$<br>P=0,024 |
| SE= erreur standard | | | | | |

Les réponses prolifératives pour chacun des quatre sous-ensembles de lymphocytes s'accordent avec celles observées pour les PBMC totales. Lors de l'exposition à l'antigène de la grippe, on observe des variations significatives dans le groupe ACTIMEL pour les quatre sous-ensembles de lymphocytes. Ces variations peuvent être représentées comme une fonction quadratique du temps.

De faibles variations ont été observées pour les quatre sous-ensembles dans le groupe placebo.

En outre, les différences entre les deux groupes étaient significatives pour tous les sous-ensembles de lymphocytes.

**CONCLUSION**

Ces résultats montrent pour les 3 antigènes testés, une augmentation de la réponse proliférative induite par un rappel antigénique plus importante dans le groupe ACTIMEL que dans le groupe placebo. Dans le cas de l'antigène de la grippe, l'augmentation dans le groupe ACTIMEL est statistiquement significative par rapport à celle observée dans le groupe placebo, aussi bien pour les PBMC totales que pour chacun des sous-ensembles cellulaires T CD3+CD4+, CD3+CD8+, CD3+CD25+ et CD3+CD45+.

Il apparaît donc que la consommation d'ACTIMEL a induit chez les sujets concernés un amorçage immunologique in vivo, qui a entraîné une réponse *ex vivo* de leurs cellules T aux antigènes microbiens et notamment aux antigènes de la grippe meilleure que celle observée dans le groupe placebo.

Cette augmentation de la réponse est en corrélation avec la fréquence et le statut d'activation des cellules T CD3[+] et de leurs sous-ensembles chez les sujets du groupe ACTIMEL.

En outre, la corrélation positive avec la présence du marqueur d'activation CD25 suggère l'intervention d'une activation *in vivo* de cellules T spécifiques de la grippe exprimant les récepteurs CD25 IL-2, et capable de répondre plus efficacement aux antigènes de la grippe. L'analyse des sous-ensembles de cellules T suggère également que la réponse proliférative implique probablement à la fois le CD4[+] et le sous-ensemble CD8[+] répondant à l'antigène de la grippe. L'importance du sous-ensemble T CD3[+]/CD8[+] dans l'analyse de la prolifération suggère la possibilité d'un amorçage *in vivo* du pool de cellules cytotoxiques CD8+ spécifiques de la grippe.

**Revendications**

1.  Utilisation d'une souche bactérienne de l'espèce *L. casei* pour la préparation d'une composition administrable par voie orale pour renforcer une réponse immunitaire systémique spécifique vis-à-vis d'un micro-organisme pathogène des voies respiratoires.

2.  Utilisation selon la revendication 1, **caractérisée en ce que** ledit micro-organisme pathogène est un virus choisi parmi les rhinovirus, le virus respiratoire syncitial, et les myxovirus.

3.  Utilisation selon la revendication 2, **caractérisée en ce que** ledit virus est un virus de la grippe.

4.  Utilisation selon une quelconque des revendications 1 à 3, **caractérisé en ce que** ladite souche de *L. casei* est la souche CNCM I-1518.

5.  Utilisation selon une quelconque des revendications 1 à 4, **caractérisée en ce que** ladite composition est sous la forme d'un aliment ou d'un complément alimentaire.

6.  Utilisation selon une quelconque des revendications 1 à 5, **caractérisée en ce que** ladite composition est sous la forme d'un produit laitier fermenté.


**Claims**

1.  Use of a bacterial strain of the species *L. casei* for the preparation of an orally administrable composition to enhance a systemic immune response towards a pathogenic microorganism of the respiratory tract.

2.  Use according to claim 1, **characterised in that** said pathogenic microorganism is a virus selected from among rhinoviruses, the syncitial respiratory virus and myxoviruses.

3.  Use according to claim 2, **characterised in that** said virus is a flu virus.

4.  Use according to any one of claims 1 to 3, **characterised in that** said strain of *L. casei* is strain CNCM I-1518.

5.  Use according to any one of claims 1 to 4, **characterised in that** said composition assumes the form of a food or food supplement.

6.  Use according to any one of claims 1 to 5, **characterised in that** said composition assumes the form of a fermented milk product.


**Patentansprüche**

1.  Verwendung eines Bakterienstamms der Art *L. casei* zur Herstellung einer auf oralem Weg verabreichbaren Zusammensetzung zur Stärkung einer spezifischen systemischen Immunreaktion gegenüber einem pathogenen Mikroorganismus der Atemwege.

2.  Verwendung nach Anspruch 1, **dadurch gekennzeichnet, dass** der pathogene Mikroorganismus ein Virus ist, der aus Rhinoviren, Syncytialatemwegsviren und Myxoviren ausgewählt ist.

3.  Verwendung nach Anspruch 2, **dadurch gekennzeichnet, dass** der Virus ein Grippevirus ist.

4.  Verwendung nach einem der Ansprüche 1 bis 3, **dadurch gekennzeichnet, dass** der Stamm von *L. casei* der Stamm CNCM I-1518 ist.

5.  Verwendung nach einem der Ansprüche 1 bis 4, **dadurch gekennzeichnet, dass** die Zusammensetzung in Form eines Nahrungsmittels oder Nahrungsergänzungsmittels vorliegt.

6.  Verwendung nach einem der Ansprüche 1 bis 5, **dadurch gekennzeichnet, dass** die Zusammensetzung in Form

**EP 1 283 714 B1**

eines fermentierten Milchprodukts vorliegt.

**11**

a. Candida          b. Tétanos          c. Grippe

FIGURE 1

FIGURE 2